# EUROPEAN PATENT APPLICATION

(11) **EP 4 821 943 A1**
(43) Date of publication of application: **14.10.2026**
(21) Application number: 25170232.0
(22) Date of filing: 11.04.2025
(51) Int. Cl.: C07K 14/435, A61K 38/00, A61K 38/17, A61P 31/04, C07K 14/47

(54) **PEPTIDES WITH ANTIMICROBIAL ACTIVITY AGAINST GRAM + AND GRAM PATHOGENS**

(71) Applicant: Politechnika Slaska w Gliwicach, 44-100 Gliwice (PL); Tübitak Marmara Research Center (Tübitak MRC), 41470 Gebze Koaceli (TR)
(72) Inventor: Gora, Artur Wiktor, 43-300 Bielsko-biala (PL); Jandoo, Jaspreet Kaur, 44-100 Gliwice (PL); Kovalenko, Oksana Petrivna, 44-100 Gliwice (PL); Alam, Md. Sarfaraz, 44-100 Gliwice (PL); Mitusinska, Karolina, 44-122 Gliwice (PL); Szawara, Marzena Maria, 44-105 Gliwice (PL); Raczynska, Agata Danuta, 44-100 Gliwice (PL); Chyski, Michal Pawel, 41-933 Bytom (PL); Papaj, Katarzyna Joanna, 42-300 Myszkow (PL); Hocaoglu, Selda Murat, 41400 Gebze/Kocaeli (TR); Usluer Güçlü, Gülsima Dilek, 41420 Gebze/Kocaeli (TR); Mumcu, Hande, 34668 Üsküdar/ stanbul (TR); Karacan, Ilker, 34774 Ümraniye/ stanbul (TR); Kayrav, Aycan, 34303 Küçükçekmece/ stanbul (TR); Aksu, Burak, 34912 Pendik/ stanbul (TR); Keles , Irem, 08000 Merkez/Artvin (TR); Yigit, Begüm, 55270 Atakum/Samsun (TR); Civelek, Kadircan, 24180 Merkez/Erzincan (TR); Öktay, Hande, 41170 Kartepe/Kocaeli (TR); Karagüler, Nevin Gül, 34457 Sar yer/ stanbul (TR)
(74) Representative: AOMB Polska Sp. z.o.o.

(57) **Abstract**

The invention relates to a peptides with antimicrobial activity against Gram + and Gram - pathogens, wherein peptide with SEQ ID NO:1 demonstrates broad-spectrum activity against *E*. *coli, S*. *aureus,* and *S*. *epidermidis;* peptide with SEQ ID NO:2 demonstrates broad-spectrum activity against *E*. *coli and S*. *aureus;* peptide with SEQ ID NO:3 demonstrates broad-spectrum activity against *S*. *epidermidis,* peptide with SEQ ID NO:4 demonstrates narrow-spectrum activity against *E*. *coli* and *P. aeruginosa,* and peptide SEQ ID NO:5 demonstrates narrow-spectrum activity against *P. aeruginosa* and *E*. *coli.*

## Description

### Field of the invention

The present invention relates to antimicrobial peptides. In particular, the invention discloses five peptides having antimicrobial activity against pathogens affecting human health, making it a potential alternative to or complement to antibiotics.

### Background art

Antibiotic resistance is considered one of the greatest health challenges of our time. It is a global issue, that not only affects developing and underdeveloped countries but also has an impact on the world at large (Llor & Bjerrum, 2014). While resistance to antimicrobials is a natural part of the evolutionary process for microorganisms, the improper and indiscriminate use of antibiotics-such as using the wrong antibiotics, incorrect dosing, inappropriate methods of administration, and improper duration of use-significantly accelerates the development of resistance in microorganisms (Andersson et al., 2020). As a result, the effectiveness of treatments for infectious diseases such as urinary tract infections, tuberculosis, sepsis, gonorrhoea, and food poisoning is progressively diminishing. Furthermore, antibiotic resistance poses a significant threat to patients undergoing surgeries, organ transplants, and chemotherapy, as they are at heightened risk of severe infections (WHO, 2018).

Antimicrobial peptides have gained significant attention in recent years as promising alternatives to antibiotics, owing to their proteinaceous structures, narrow spectra of activity, and minimal interaction with host organisms.

Peptide-based drugs account for only a small fraction of the molecules approved by the FDA. Of over 3,000 antimicrobial peptides, only 7 have received FDA approval, and all of these are derived from soil-dwelling organisms. These FDA-approved peptides are typically used in treatments for bacterial skin infections, conjunctivitis, and wounds.

The closest prior art includes known antimicrobial peptides; however, it does not disclose the specific amino acid sequences described in this invention nor their confirmed activity against selected pathogens such as E. *coli,* S. *aureus,* S. *epidermidis,* and *P. aeruginosa.*

During the search of the sequence database, it was found that there are sequences similar or identical to one of the claimed in this invention, i.e. hypothetical protein CML47_07075 *(Paracoccaceae bacterium)* or *Chrysochloris asiatica.*

The applied modifications to the sequences (e.g. scolopendin-1 variants or ubiquitin-like peptides) yield compounds with improved or selective biological activity, while retaining compatibility with chemical synthesis or recombinant expression. These effects could not have been predicted based on existing solutions and were achieved through experimental insight and a creative approach to selecting specific sequence modifications, as described in this invention.

The technical problem addressed by this invention is the growing prevalence of antimicrobial resistance, driven by the inappropriate and excessive use of antibiotics. Such misuse accelerates the development and spread of resistant pathogens, diminishing the effectiveness of existing treatments. Once a new antibiotic is introduced into an environment containing microorganisms, those with resistance-or the ability to acquire it-can survive and proliferate, contributing to the rise of resistant strains.

This invention focuses on peptide-based molecules with antimicrobial activity as an alternative to conventional antibiotics. These peptides are effective against both Gram-positive (Gram +) and Gram-negative (Gram -) pathogens that impact human health. Owing to their peptide structure, they are suitable for chemical synthesis or recombinant expression, and they offer a promising solution to combat resistant infections. By providing activity against clinically relevant pathogens, the invention has the potential to serve as either an alternative to or a complement for current antibiotic therapies.

### The subject matter of the invention

The invention relates to a peptide with antimicrobial activity against Gram + and Gram - pathogens selected from the group comprising the amino sequence:
A) SEQ ID NO:1
B) SEQ ID NO:2
C) SEQ ID NO:3
D) SEQ ID NO:4
E) SEQ ID NO:5

wherein SEQ ID NO:1 and SEQ ID NO:2 are considered as active variants of scolopendin 1,
wherein SEQ ID NO:3, SEQ ID NO:4 and SEQ ID NO:5 are ubiquitin-like peptides.
wherein SEQ ID NO:1 demonstrates broad-spectrum activity against *E. coli, S. aureus,* and *S. epidermidis;* SEQ ID NO:2 demonstrates broad-spectrum activity against E. *coli and S. aureus;* SEQ ID NO:3 demonstrates activity against *S. epidermidis,* SEQ ID NO:4 demonstrates activity against *E. coli* and *P. aeruginosa,* and SEQ ID NO:5 demonstrates broad-spectrum activity against *P. aeruginosa* and *E. coli.*

### Brief description of the figures

The invention will be further elucidated in the following non-limiting exemplary embodiments, with reference to the accompanying figures, wherein:
Fig. 1 shows pictures of plates showing antimicrobial activity of selected peptides. (A) Representative radial diffusion assay plates showing zones of inhibition (ZOI) for peptides A) N39 B) J29 C) M02, D) P81, and E) G30 against a lawn growth of 'Gram negative' *(E. coli, P. aeruginosa) and* Gram Positive *(S. aureus, S. epidermidis).* Dash-line square shows the peptide demonstrating the zone of inhibition by the indicated bacterial strain.
Fig. 2 shows an antimicrobial cell viability activity in microtitre broth G30.
Fig. 3 shows predicted 3D structures of the antimicrobial peptides - A - N39; B - J29; C - G30; D - P81; E - M02.
Fig. 4 shows purification of P81 using Glutathione agarose 4B beads.
Fig. 5 shows SDS-analysis of the M02.1 peptide purification.

### Detailed description of the invention

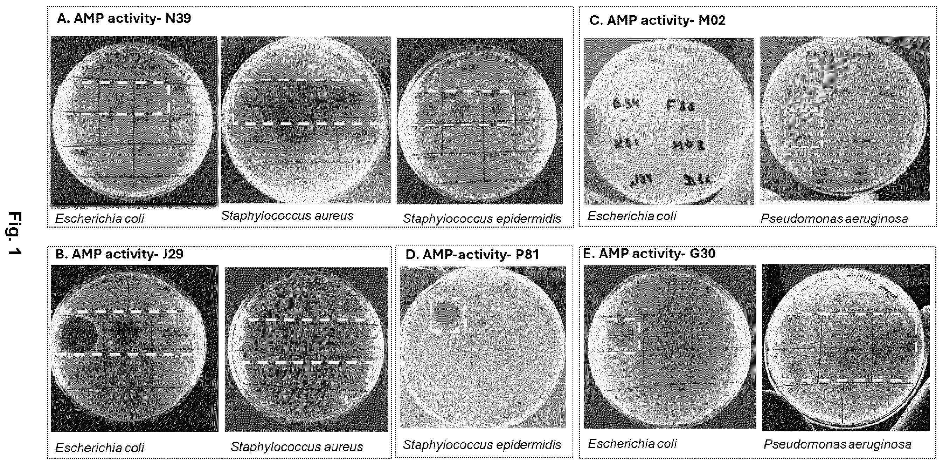

Among 16 peptides screened, five (N39, J29, G30, P81, M02) exhibited antimicrobial activity in the radial diffusion assay. Peptide N39 (APO02453) (SEQ ID NO:1) demonstrated broad-spectrum activity against *E. coli, S. aureus,* and *S. epidermidis,* while J29 (APO02453) (SEQ ID NO:2) was active against *E. coli* and *S. aureus.* Peptides annotated to DRAMP18492 (G30, P81, M02) showed strain-specific activity: P81 (SEQ ID NO:3) against *S. epidermidis,* G30 (SEQ ID NO:4) against *E. coli* and *P. aeruginosa,* and M02 (SEQ ID NO:5) against *P. aeruginosa* and *E. coli.* Antimicrobial activity of selected peptides is shown on Figure 1 and in Table 1.

**N39 and J29 that can be considered as active variants of scolopendin 1:**
N39 - MIGQKLGPYEILSSIGSGGMGEVWKARDTRLGRIVAIKKVILAN (SEQ ID NO:1)
J29 - MIGKTISHYKILSKLGEGGMGTVYKAEDTKLKRMVALKLLS (SEQ ID NO:2)

### P81, G30, M02 ubiquitin-like peptides

P81 - MVIYPSNKKKNLKTCCVIIKKEMQIFVKTLTGKTITLDVESSDTIDNVKQKIQDKEG (SEQ ID NO:3)
G30 - 
MEPSDTIENVKSKIQDKEGIPPDQQRLIFAGKQLEDGRTLSDYNIQKEATLHLVLRLR
GGC (SEQ ID NO:4)
M02 - MAILAKQQRLIFGSEQMEDASALSDNNIQNESTIQLMHGLH (SEQ ID NO:5)

**Table 1. Antimicrobial Activity of Selected Peptides**

| **Peptide** | **APO02453/DRAMP18492 Annotation** | **Gram *-*** | | **Gram+** | |
|---|---|---|---|---|---|
| | | ***E. coli*** | ***P. aeruginosa*** | ***S. aureus*** | ***S. epidermidis*** |
| N39 | APO02453 | + | - | + | + |
| J29 | APO02453 | + | - | + | X |
| P81 | DRAMP18492 | - | - | - | + |
| G30 | DRAMP18492 | + | + | - | - |
| M02 | DRAMP18492 | + | + | - | - |

| | | | | | |
|---|---|---|---|---|---|
| Key: "+" = Activity observed; "-" = No activity observed. "X"= Not tested | | | | | |

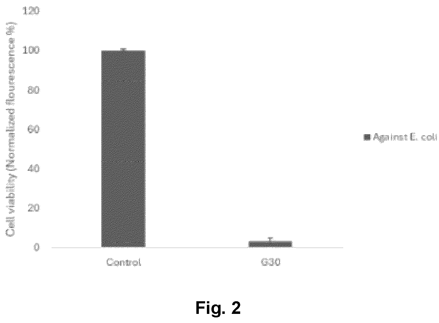

Figure 2 shows that G30 demonstrated antimicrobial activity against *E. coli* in liquid culture by using micro-dilution broth assay technique for cell viability.

### Description of each peptide

### N39

### N39 - 44 aa long peptide with unique sequence:

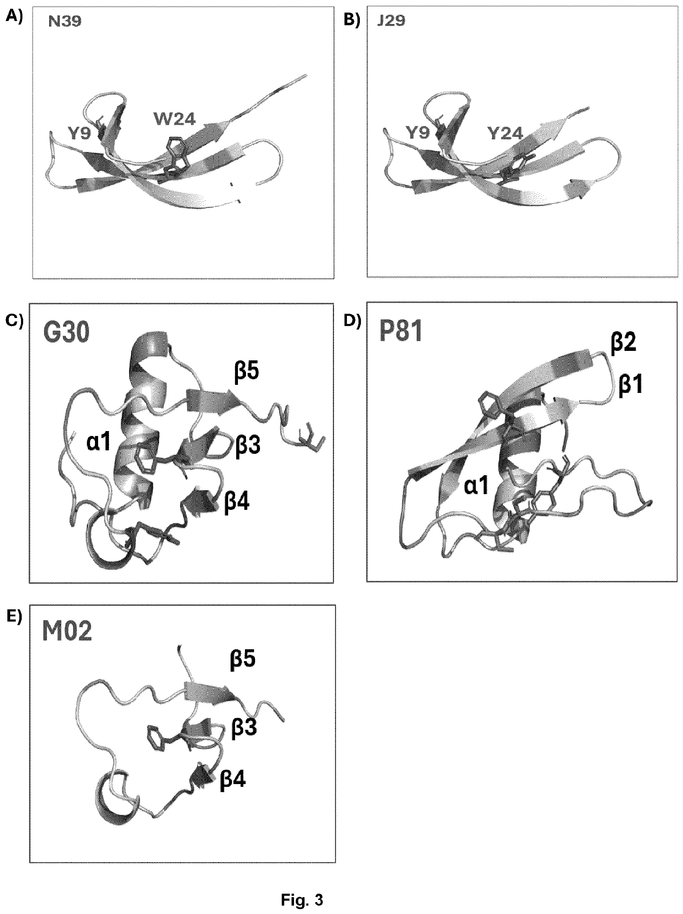

MIGQKLGPYEILSSIGSGGMGEVWKARDTRLGRIVAIKKVILAN (SEQ ID NO:1) and predicted 3D structure resembling β-sheet build from 3 strands (Fig. 3, A). Molecular weight: 4727,66 Da; charge at pH 7.4 - +3.61.

### Synthesis of N39:

The peptide N39 is commercially synthesized by Gene Script using solid-phase peptide synthesis, a method that involves assembling the peptide on a solid support, followed by cleavage and purification. This peptide was synthesized in a high-purity product of over 95%.

**Activity assay.** A single colony of reference bacteria was inoculated in 3 mL of tryptic soy broth (TSB) for overnight incubation at 37 °C in a shaking incubator maintained at 280 rpm. The optical density of the overnight cultures was normalized to a 0.5 McFarland Standard (equivalent to 1.5 × 10⁴ CFU/mL) using a densitometer in Mueller-Hinton II (MHII) broth. Subsequently, 10 µL of the standardized bacterial suspension was inoculated into 10 mL of autoclaved and pre-cooled MHII broth containing 0.75% (w/v) agarose (low EEO). This mixture was gently mixed and dispensed into sterile Petri dishes. Then, the plates were briefly dried (10-15 min). Each purified antimicrobial peptide (AMP) was applied as a 20 µL spot onto the surface of the MHII agarose. The plates were incubated overnight at 25 °C and then visually inspected for the formation of inhibition halos.

Peptide was tested for its activity against *Escherichia coli ATCC* 25922, *Pseudomonas aeruginosa ATCC 27853, Staphylococcus aureus ATCC* 25923, and Staphylococcus *epidermidis ATCC* 12228. Activity tests were performed in triplicate for each target bacterial strain.

**Result:** N39 demonstrated antimicrobial activity against Gram -ve **E**. *Coli,* and Gram +ve, *S*. *aureus and S*. *Epidermidis.* This result suggests its antimicrobial efficacy against broad spectrum microorganisms.

### J29

J29 - 41 aa long peptide with unique sequence MIGKTISHYKILSKLGEGGMGTVYKAEDTKLKRMVALKLLS (SEQ ID NO:2) and predicted 3D structure resembling β-sheet build from 3 strands (Fig. 3, B). Molecular weight: 4510.48 Da; charge at pH 7.4: +4.289.

### J29 Synthesis and activity assay.

### Synthesis of J29

The peptide J29 is commercially synthesized by ProteoGenix using solid-phase peptide synthesis, a method that involves assembling the peptide on a solid support, followed by cleavage and purification. This peptide was synthesized in a high-purity product of over 90%.

**Activity assay.** This part of an experiment was performed in the exact same way as for N39.

**Result:** J29 demonstrated antimicrobial activity against Gram -ve *E*. *coli* and Gram +ve *S*. *aureus.* This result suggests antimicrobial efficacy of the J29 peptide against broad spectrum microorganisms.

### P81

### P81 - 57 aa long peptides with unique sequence:

MVIYPSNKKKNLKTCCVIIKKEMQIFVKTLTGKTITLDVESSDTIDNVKQKIQDKEG (SEQ ID NO: 3) and predicted 3D structure containing one α-helix and one β-sheet (build from 2 strands) connected by loops, (Fig. 3, C). Molecular weight: 6487.69 , charge at pH 7.4: +3.001.

### P81 purification and activity assay.

### P81 Cloning

The gene sequence for P81 was engineered by reverse translation and tailored for optimal expression in *Escherichia coli.* The P81 gene, commercially synthesised by BioCat GmbH (Germany), was incorporated into the pGEX-6P-1 expression vector between the Pasl and Notl restriction sites. This recombinant construct was subsequently introduced into *E. coli*Origami B (DE3 pLysS) cells to enable protein production.

### Recombinant Protein Expression

A single colony from transformed cells was inoculated in 20 ml of starter culture supplemented with 100 µg/ml ampicillin. 10 ml of starter culture was added to 1 liter of LB media and allowed to grow until optical density reached (OD ₆₀₀) 0.6-0.8. Recombinant protein synthesis was induced with 0.5 mM isopropyl-β-D-thiogalactopyranoside (IPTG) at 18°C for 12-16 hrs. Afterwards the cells were then harvested by centrifugation and stored at -80°C until further use.

### Peptide Purification

The cells were resuspended in 20-25 mL of lysis buffer, which is phosphate-buffered saline (PBS) at pH 7.3, supplemented with 5 mM DTT, 1 mM EDTA, one tablet of EDTA-free protease inhibitor cocktail, and a pinch of DNase I. The cells were lysed by sonication (amplitude: 70%, Rounds number: 10, pulse ON: 10 s, pulse OFF: 20 s). The lysed cells were clarified by centrifugation at 18000 xg for 60 minutes at 4°C. This AMP was purified by chromatography on Glutathione Agarose 4B (Macherey-Nagel) following the manufacturer's recommendations for gravity flow purification. 2 mL of Glutathione Agarose beads were equilibrated with 10 CV of 1x PBS (pH 7.4) and 5 CV of lysis buffer. The cleared lysate was then incubated with the beads for binding for 2 hours at 4°C. The Agarose beads were first washed with 5 CV of Buffer A (PBS pH 7.4, 1% Triton X100, 5 mM DTT, 1 mM EDTA), then 10 CV with Buffer B (PBS pH 7.4, 5 mM DTT, 1 mM EDTA), and finally equilibrated the bound GST-AMP with 10 CV Buffer C (Cleavage Buffer: 50 mM Tris pH 8.0, 100 mM NaCl, 1 mM DTT, and 1 mM EDTA). The Glutathione Agarose beads were treated with 5 ml of Buffer C containing 500 µg of precision protease and incubated overnight in a fridge at 4°C. The cleaved AMPs were collected in the flow through , and the bound GST and GST-PreScission Protease were eluted with Buffer D (50 mM Tris pH 8.0+ 10 mM reduced glutathione). The AMPs purified from the Glutathione Agarose 4B column contain some impurities together with GST. However, due to the extreme aggregation propensity of this peptide, further improvement to achieve a pure peptide beyond this step is not feasible. Additionally, attempts to synthesize this peptide chemically were unsuccessful, likely due to its complex sequence, aggregation propensity, and hydrophobic nature, making recombinant expression and partial purification the most feasible option. The resulting peptide retained an N-terminal GPLG sequence, a remnant of the PreScission Protease cleavage site, appended to the native P81 sequence.

### GPLG

### MVIYPSNKKKNLKTCCVIIKKEMQIFVKTLTGKTITLDVESSDTIDNVKQKIQDKEG

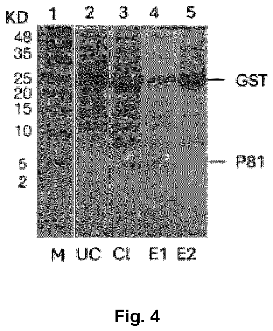

Fig. 4 shows purification of P81. Lane 1 displays the molecular weight marker. Lane 2 shows the uncleaved fraction, while Lane 3 presents the cleaved peptide. Lane 4 shows the eluted peptide (E1) of P81 after cleavage from the column. Finally, Lane 5 illustrates the eluted GST (E2) after cleavage. Star represents the peptide P81.

**Activity assay.** This part of an experiment was performed in the exact same way as for N39 and J29.

**Result:** The peptide P81 demonstrated antimicrobial activity against Staphylococcus *epidermidis ATCC* 12228 suggesting its potent activity against Gram +ve bacteria.

### G30

### G30 - 61 aa long peptide with unique sequence:

MEPSDTIENVKSKIQDKEGIPPDQQRLIFAGKQLEDGRTLSDYNIQKEATLHLVLRLR GGC (SEQ ID NO:4) and predicted 3D structure containing one α-helix and one β-sheet (build from 3 strands) connected by loops. Molecular weight: 6894.84 Da, charge at pH 7.4: -1.719.

### G30 purification and activity assay

### Synthesis of G30

The peptide G30 is commercially synthesized by ProteoGenix using solid-phase peptide synthesis, a method that involves assembling the peptide on a solid support, followed by cleavage and purification. This peptide was synthesized in a high-purity product of over 90%.

**Activity assay.** This part of an experiment was performed in the exact same way as for N39, J29 and P81.

**Micro-dilution broth assay.** A single colony of reference bacteria was inoculated in 3 mL of tryptic soy broth (TSB) and incubated overnight at 37 °C in a shaking incubator set to 280 rpm. The optical density of the overnight culture was adjusted to a range of 0.001 to 0.01 at 600 nm using a spectrophotometer in Mueller-Hinton II (MHII) broth. In a 96-well plate, 20 µL of synthesised peptide was added to each well, along with 80 µL of MHII, and a two-fold serial dilution was prepared. Next, 5 µL of normalized target bacteria was seeded into each well. For the negative control, bacteria were seeded with broth only, and just broth was used as a blank control. The plate was then incubated at 25 °C overnight under shaking conditions at 300 RPM. After 16-20 hours of incubation, 10 µL of 1x resazurin-based PrestoBlue^{™} (Thermo Fisher Scientific) cell viability reagent was added to each well, and the plate was incubated again under the same conditions. Readings were taken after 30 minutes of incubation. Measurements were recorded at absorbance values of 600 nm and 570 nm (the reference absorbance for the reagent), while excitation and emission fluorescence were measured at 560 nm and 590 nm, respectively, as outlined by the manufacturer. Peptide was tested for its activity against *Escherichia coli ATCC 25922.* Activity tests were performed in triplicates for each target bacterial strain.

**Result:** G30 peptide was tested for its activity against *Escherichia coli ATCC 25922.* We observed wells with no visible growth against *E*. *coli* strains. Additionally, the radial diffusion agar plate assay demonstrated significant antimicrobial activity against E. *coli,* but a weak activity against *P. aeruginosa.* These results suggest that the G30 has potency against Gram-negative bacteria.

### M02

### M02 - 41 aa long peptide with unique sequence:

MAILAKQQRLIFGSEQMEDASALSDNNIQNESTIQLMHGLH (SEQ ID NO:5) and predicted 3D structure resembling β-sheet build from 3 strands. Molecular weight - 4584.19 Da, charge at pH 7.5 - (-3.16).

### M02 purification and activity assay

The sequence was selected from a metagenomic library as a ubiquitin-like peptide. The reference protein was DRAMP18492 (Seo et al., 2013). Pairwise alignment demonstrated 33.8% similarity between M02 and the reference sequences (EMBOSS Needle; Madeira et al, 2024).

**M02 cloning.** The M02 sequence was back-translated to the DNA sequence and optimised for translation in *E.coli* cells. M02 encoding gene was commercially synthesised by BioCat GmbH (Germany) and cloned into pGEX-6P-1 between *Pasl* and *Notl* sites. The resulting plasmid was transformed into BL21 (DE3) *E.coli* cells.

**Recombinant protein expression.** The cells containing the designed plasmid were grown in 250 mL of Lysogeny Broth containing ampicillin (100 mg/L) at 37 °C with shaking until the optical density at 600 nm reached 0.6-0.7. Protein expression was induced by addition of IPTG to the final concentration of 0.5 mM, and the cells were further incubated at 37 °C for 4 hours. Then, the induced cells were harvested by centrifugation and stored at -80 °C.

**Peptide purification.** Cell lysis was performed in 3 mL of Buffer A (phosphate-buffered saline, pH 7.4, 100 mg/L MgSO₄*6H₂O, 100 mg/L CaCl₂ (water-free)) supplemented with 1 mg/mL lysozyme. After incubation for 30 min on ice and sonication, the cell debris was separated by centrifugation. The clarified cell lysate containing GST-tagged M02 peptide was mixed with 1 mL (settled volume) of Protino^{®} Glutathione Agarose 4B (Macherey-Nagel GmbH) in a closed column and incubated with gentle agitation at 0 °C for 4 hours. After washing the column with 30 mL of Buffer A, the buffer was changed to Buffer B (50 mM Tris-HCl, pH 8.0, 100 mM NaCl, 1 mM DTT, 1 mM EDTA). On-column GST-tag removal was performed by incubation with 0.4 mg/mL of in-house purified GST-tagged PreScission Protease at 0 °C overnight with gentle agitation. Then, the flow-through fraction containing the eluted M02 peptide was collected and stored at -20 °C. The purity of the peptide was above 90% judging from Tris-Tricine SDS-PAGE analysis. The resulting peptide, named M02.1 contained an additional GPLG sequence (coming from PreScission Protease cutting site) at the N-termini of the original M02 sequence.

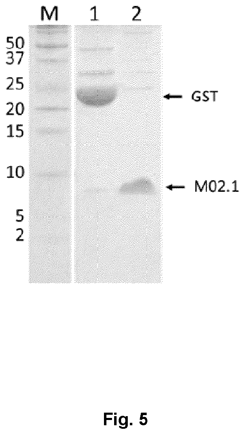

Fig. 5 shows SDS-analysis of the M02.1 peptide purification. Line 1 - proteins bound to Glutathione-Agarose 4B after incubation with PreScission Protease. Line 2 - eluate. M - Precision Plus Protein Dual Xtra Prestained Protein Standards (Bio-Rad).

**Activity assay.** Target cells were grown in 3 mL of Mueller Hinton Broth at 37 °C overnight. The optical density of the overnight cell culture was normalized to a 0.5 McFarland Standard. 1mL of the normalized cell culture was applied onto a Mueller Hinton agar (MHA) plate, immediately distributed and aspirated. Then, the MHA was briefly dried (10-15 min), and 5 µL of the purified peptide were spotted onto the surface of the MHA. The plates were incubated for 16-23 hours at 29-30 °C and then visually inspected for a halo formation.

M02.1 peptide was tested for its activity against *Escherichia coli ATCC 25922, Pseudomonas aeruginosa ATCC* 27853, *Staphylococcus aureus ATCC* 25923 and *Staphylococcus epidermidis ATCC* 12228. Activity tests were performed in triplicates for each target bacterial strain. Observed halos formed on *P.aeruginosa* and *E.coli* lawns suggested M02.1 potency against Gram-negative bacterial strains. In contrast, M02.1 did not demonstrate antibacterial activity toward Gram-positive S. *aureus* and *S. epidermidis* strains.

The narrow distribution of DRAMP18492 sequences (enriched in IK1 and MD19A sampling sites) contrasts with that of other ubiquitin-like families. This suggests that there may be localized evolutionary pressure favouring specific truncations. The retention of α1/β-sheet motifs in active peptides (G30, P81, M02) indicates that these regions are likely under positive selection, potentially acting as a host defence strategy in oyster gills (Mitta et al., 2000). Additionally, the identification of N39 and J29 as active variants of scolopendin 1 highlights the potential of sequence truncation in modulating antimicrobial efficacy.

## Claims

1. Peptide with antimicrobial activity against Gram + and Gram - pathogens selected from the group comprising the amino sequence:
A) SEQ ID NO:1
B) SEQ ID NO:2
C) SEQ ID NO:3
D) SEQ ID NO:4
E) SEQ ID NO:5

2. The peptide according to claim 1, wherein SEQ ID NO:1 and SEQ ID NO:2 are considered as active variants of scolopendin 1.

3. The peptide according to claims 1 and 2, wherein SEQ ID NO:3, SEQ ID NO:4 and SEQ ID NO:5 are ubiquitin-like peptides.

4. The peptide according to claims 1-3, wherein SEQ ID NO:1 demonstrates broad-spectrum activity against *E. coli, S. aureus,* and *S. epidermidis;* SEQ ID NO:2 demonstrates broad-spectrum activity against *E. coli and S. aureus;* SEQ ID NO:3 demonstrates activity against *S. epidermidis,* SEQ ID NO:4 demonstrates activity against *E. coli* and *P. aeruginosa,* and SEQ ID NO:5 demonstrates narrow-spectrum activity against *P. aeruginosa* and *E. coli.*
